# FASCICULE DE BREVET EUROPEEN

(11) **EP 3 089 751 B1**
(45) Date de publication et mention de la délivrance du brevet: **08.05.2019**
(21) Numéro de dépôt: 14814911.5
(22) Date de dépôt: 20.11.2014
(51) Int. Cl.: A61K 36/185, A61K 36/73, A61P 17/00, A61P 31/04, A61P 31/10, A61K 8/97, A61Q 5/00, A61Q 19/00, A61K 9/00

(54) **COMPOSITION TOPIQUE COMPRENANT DES EXTRAITS DE BOLDO ET DE REINE DES PRES, DESTINEE A UN ANIMAL, ET SES APPLICATIONS**
FÜR EIN TIER BESTIMMTE, TOPISCHE ZUSAMMENSETZUNG MIT EXTRAKTEN AUS BOLDO UND MADESÜSS UND VERWENDUNGEN DAVON
TOPICAL COMPOSITION COMPRISING EXTRACTS OF BOLDO AND OF MEADOWSWEET, INTENDED FOR AN ANIMAL, AND USES THEREOF

(30) Priorité: 31.12.2013 FR 1303124
(43) Date de publication de la demande: 09.11.2016
(73) Titulaire: Virbac SA, 06510 Carros (FR)
(72) Inventeur: GATTO Hugues, F-06570 Saint Paul de Vence (FR)
(74) Mandataire: Macquet, Christophe
(86) Numéro de dépôt international: PCT/FR2014/000249
(87) Numéro de publication internationale: WO 2015/101722

(56) Documents cités:
- EP-A2- 2 322 138
- FRANE BANOVIC ET AL: "In vitro comparison of the effectiveness of polihexanide and chlorhexidine against canine isolates of Staphylococcus pseudintermedius , Pseudomonas aeruginosa and Malassezia pachydermatis", VETERINARY DERMATOLOGY, vol. 24, no. 4, 21 juin 2013 (2013-06-21), pages 409-e89, XP055129333, ISSN: 0959-4493, DOI: 10.1111/vde.12048
- YILDIRIM A B ET AL: "Biological activities of meadowsweet (Filipendula ulmaria (L.) Maxim)", PLANTA MEDICA, THIEME VERLAG, DE, vol. 75, no. 9, 1 juillet 2009 (2009-07-01), page 1066, XP009179137, ISSN: 0032-0943, DOI: 10.1055/S-0029-1234950
- VILA R ET AL: "Composition and antimicrobial activity of the essential oil of Peumus boldus leaves.", PLANTA MEDICA MAR 1999, vol. 65, no. 2, mars 1999 (1999-03), pages 178-179, XP002727355, ISSN: 0032-0943
- HMPC (Committee on Herbal Medicinal Products): "ASSESSMENT REPORT ON PEUMUS BOLDUS MOLINA, FOLIUM", EMEA , 14 janvier 2009 (2009-01-14), XP055129349, Extrait de l'Internet: URL:http://www.ema.europa.eu/docs/en_GB/do cument_library/Herbal_-_HMPC_assessment_re port/2009/12/WC500018102.pdf [extrait le 2014-07-16]
- HMPC (Committee on Herbal Medicinal Products): "Assessment report on Filipendula ulmaria (L.) Maxim., herba and Filipendula ulmaria (L.) Maxim., flos", EMEA , 12 juillet 2011 (2011-07-12), XP055129352, Extrait de l'Internet: URL:http://www.ema.europa.eu/docs/en_GB/do cument_library/Herbal_-_HMPC_assessment_re port/2011/09/WC500115355.pdf [extrait le 2014-07-16]

## Description

La présente invention concerne une composition pour administration topique destinée à un animal, comprenant des extraits de plantes stimulant la synthèse cellulaire de peptides antimicrobiens, notamment pour son utilisation dans la prévention, le traitement ou l'aide au contrôle des infections microbiennes. Plus particulièrement, l'invention concerne une composition pour administration topique destinée à un animal domestique comprenant des extraits de boldo, *Peumus boldus,* et des extraits de reine des prés *Spiraea ulmaria.*

La fonction principale de la peau consiste à assurer la protection entre le monde terrestre hostile et l'intérieur de l'organisme. Cette fonction défensive est rendue possible par les deux fonctions principales suivantes :
(i) une fonction de barrière épidermique. Elle est localisée sur la partie extérieure (*stratum corneum*) de la couche de la peau la plus superficielle (l'épiderme). La structure de la couche cornée peut être comparée à un mur de briques, dans lequel les briques représentent les cellules mortes (cornéocytes) et le mortier, une matrice intercellulaire organisée en structure lamellaire lipidique. Cette barrière protège l'organisme contre les agressions chimiques, microbiologiques, physiques et régule les échanges en eau entre l'organisme et l'environnement ;
(ii) le système immunitaire cutané (CIS qui vient de l'anglais « Cutaneous Immune System »). Le CIS a la capacité d'induire une réponse inflammatoire contre les agressions extérieures. Le CIS peut être divisé en 2 parties, une partie innée et une adaptative:
   - Le système immunitaire inné correspond au mécanisme de première ligne, qui est non-spécifique contre l'invasion microbienne. Dans le cas où la barrière physique est rompue, la réponse immunitaire innée est activée pour éliminer les agresseurs. Le système inné utilise des récepteurs préexistants qui peuvent se lier à des molécules et/ou à des motifs moléculaires communément trouvés sur de nombreux microbes. Toutefois, ce système immunitaire inné reste incapable de développer une mémoire des agressions et donc une protection efficace vis-à-vis des réinfections ;
   - Le système immunitaire adaptatif consiste quant à lui en une réponse immunitaire retardée et spécifique. Il est capable de reconnaître de façon plus spécifique les agressions extérieures et antigènes, de les détruire et de garder en mémoire les agressions subies.

Les kératinocytes sont des cellules constituant 90% de l'épiderme et des phanères, c'est-à-dire les ongles, cheveux, poils, plumes, et écailles. Ils jouent un rôle important dans la fonction immunitaire locale. Ils produisent en effet des interleukines et des cytokines visant à préserver l'homéostasie c'est-à-dire l'équilibre de la barrière.

Les peptides antimicrobiens du système immunitaire inné, PAMs ou AMPs pour « antimicrobial peptides » en anglais, tels que les cathélicidines ou les béta-défensines, qui sont produits par la peau, sont stockés dans les corps lamellaires et sont ensuite délivrés à la couche cornée.

Ces peptides antimicrobiens PAMs sont des petits peptides ayant entre environ 12 et 50 acides aminés. Ils sont considérés comme l'un des éléments clés du système immunitaire inné, servant à la défense des organismes multicellulaires lors d'infections microbiennes. Ils possèdent des propriétés d'inhibition directe contre les agents pathogènes microbiens. Ils s'attachent puis s'insèrent dans la bicouche phospholipidique microbienne, induisant une rupture de la membrane microbienne puis une lyse.

Il existe un grand nombre de PAMs. Les principaux PAMs connus sont les béta-défensines (BD) et les cathélicidines (Cath). Les béta-défensines et les cathélicidines ont été identifiées dans la peau (épiderme) d'un grand nombre de mammifères, notamment l'Homme, le Chien, le Chat et le Cheval. Ces PAMs sont synthétisés par les leucocytes et le tissu épithélial des muqueuses. Ils sont cependant principalement produits par les kératinocytes. Ils sont exprimés ou induit par des composantes bactériennes (lipopolysaccharides) ou par des médiateurs pro-inflammatoires (à titre d'exemple : IL-6 pour Interleukine-6, IL-8, interféron-gamma, TNF-alpha).

Les béta-défensines (BD) possèdent une activité antimicrobienne et jouent un rôle immunomodulateur (par activité chimiotactique). Chez les animaux et notamment chez le chien, les données existantes concernant l'expression de béta-défensines et notamment cBD103 sont contradictoires. Ainsi, l'expression de l'ARNm de cBD103 chez des chiens atopiques serait inférieure à l'expression de l'ARNm de cBD103 chez des chiens sains selon les études présentées par VanDamme et al., Mol Immuno 2009. Cette expression d'ARNm entre chiens atopiques et chiens sains serait similaire selon les études présentées par Léonard et al., J Innate Immunol 2012. Enfin, d'après les études de Santoro et al., Vet Derm 2013 l'expression de l'ARNm de cBD103 chez des chiens atopiques serait augmentée par rapport aux chiens sains.

De même, selon Santoro et al., Vet Derm 2013, l'expression de l'ARNm de cBD103 au niveau des lésions cutanées (sites lésionnels) serait augmentée par rapport aux sites non-lésionnels. En revanche, selon les études présentées par Léonard et al., J Innate Immunol 2012, l'expression d'ARNm de cBD103 serait sensiblement similaire entre les sites lésionnels et non-lésionnels.

Les cathélicidines (Cath) sont également des agents antimicrobiens puissants. Elles présentent une capacité intrinsèque à tuer des bactéries Gram négatives et Gram positives, des champignons et des virus. Elles favorisent également une réponse de l'hôte en déclenchant le recrutement des cellules immunocompétentes et la libération de cytokines.

Il a été montré qu'une altération de l'expression des PAMs chez des sujets atteints d'altération de la barrière cutanée (par exemple lors de lésions cutanées ou lors de dermatites, telles que la dermatite atopique canine ou féline, la dermatite à *Malassezia*, lors de pyodermites, d'otites chroniques, ou encore d'infections fongiques ou bactériennes) pouvait prédisposer l'hôte à des infections secondaires. Les niveaux d'expression de béta-défensines et de cathélicidines de chiens atopiques sont modifiés par rapport à leurs niveaux d'expression chez des chiens sains.

Les altérations de la barrière cutanée peuvent être induites par la pathologie de manière primaire, ou secondairement par le prurit. La dermatite atopique notamment, est une maladie prurigineuse chronique de la peau apparaissant dans de nombreuses espèces. Les animaux présentant une dermatite atopique ont une prédisposition génétique à développer les réactions d'allergie vis-à-vis d'antigènes environnementaux, comme le pollen ou les moisissures. Cela aboutit à la production excessive d'anticorps réaginiques (IgE). On estime qu'environ 10% des chiens souffrent d'atopie. Les lésions cutanées générées par cette maladie sont grandement aggravées par le léchage, le grattage, et les infections bactériennes. Les chiens atopiques présentent par ailleurs des taux élevés de *Staphylococcus pseudintermedius* et de *Malassezia pachydermatis.*

Compte tenu de ce qui précède, il existe donc un besoin de développer une composition pour administration topique destinée à un animal qui soit capable de stimuler l'immunité innée de la peau, en augmentant l'expression des béta-défensines et/ou des cathélicidines, de préférence chez les chiens ou les chats.

Il a été montré que, parmi 300 substances, l'extrait de boldo est la substance la plus efficace pour stimuler l'expression de la béta-défensine humaine hBD-3. En plus de son action spécifique sur hBD-3, l'extrait de boldo, constitué notamment d'un alcaloïde, la boldine, possède un spectre d'action large contre les bactéries Gram-positives et Gram-négatives. L'extrait de boldo est également connu pour être efficace contre *Staphylococcus aureus.*

Formulé à 4%, l'extrait de boldo permet notamment la réduction des imperfections de la peau chez les sujets souffrant de troubles sévères de la peau.

La demande FR2843125 décrit des compositions pharmaceutiques comprenant un principe actif capable de stimuler l'expression humaine de hBD-2 et/ou hBD-3. Parmi les actifs utilisables, on peut citer l'extrait de boldo.

La demande FR2863893 décrit quant à elle l'utilisation d'un principe actif capable de stimuler l'expression humaine de hBD-2 et/ou hBD-3 pour la fabrication d'une composition cosmétique destinée à exercer une activité bactéricide et/ou fongicide sur la peau. Parmi les actifs utilisables, on peut également citer l'extrait de boldo.

Le produit Betapur™ commercialisé par la société BASF comprend de l'extrait de boldo, *Peumus boldus,* et induit la production de béta-défensines sans engendrer d'inflammation au niveau des kératinocytes humains.

Chez l'Homme, il a également été montré que le produit Dermapur® commercialisé par la société Silab permettait de préserver l'écosystème de la peau en stimulant la synthèse cellulaire des peptides antimicrobiens de type cathélicidines. Le produit Dermapur® est une solution comprenant 1.9% d'extraits de reine des prés, *Spiraea ulmaria* (ou Meadowsweet en anglais). Cet extrait de plantes, qui est particulièrement riche en acides phénoliques, peut notamment inhiber de 92% la croissance de *Propionibacterium acnes,* et inhiber de 82% celle de *Staphylococcus aureus,* l'une des bactéries Gram-positives les plus courantes qui entraine notamment des infections cutanées chez l'Homme.

Le produit Dermapur® est connu pour être un biorégulateur de la microflore cutanée présentant une activité anti-peaux grasses et de traitement des pores. La demande FR2897778 décrit d'ailleurs des compositions comprenant de la reine des prés pour améliorer l'état des peaux à tendance grasse et/ou acnéique.

De façon similaire, il a été montré que les composés phénoliques des végétaux présentent une activité antibactérienne contre les bactéries Gram-positives, en particulier contre *Staphylococcus aureus.*

De cette manière, en stimulant les défenses antibactériennes naturelles, l'extrait de reine des prés contribue à l'homéostasie cutanée.

Bien qu'il ne soit pas possible de généraliser les travaux réalisés chez l'Homme à des travaux sur des animaux, la Demanderesse a décidé d'évaluer le potentiel de la reine des prés et du boldo sur l'expression des peptides antimicrobiens (comme la cathélicidine canine cCath et la béta-défensine canine cBD103) à la surface de la peau des animaux et de leur possible intérêt pour traiter les troubles de la peau des animaux (tels que la dermatite atopique canine).

La solution au problème posé a pour objet une composition topique pour animaux, caractérisée en ce qu'elle comprend, dans un milieu physiologiquement acceptable, des extraits de boldo *Peumus boldus* et des extraits de reine des prés *Spiraea ulmaria.*

L'invention concerne donc notamment une composition vétérinaire, c'est-à-dire destinée aux soins des animaux, ces soins pouvant être thérapeutiques ou non-thérapeutiques.

De façon surprenante, comme cela est illustré notamment à l'exemple 1 ci-dessous, la Demanderesse a pu mettre en évidence que l'association d'extraits de boldo *Peumus boldus* et d'extraits de reine des prés *Spiraea ulmaria* est particulièrement efficace dans le développement d'une stratégie visant à réduire l'apparition et la progression d'infections microbiennes, et ainsi prévenir, traiter ou encore aider à contrôler le développement de ces infections microbiennes.

Par ailleurs, ces extraits de plantes, pris seuls ou en mélange, n'entrainent pas l'augmentation de la chimiokine IL-8 ou de la cytokine TNF-α. Autrement dit, aucun des deux extraits, pris seuls ou en combinaison n'entraîne de réponse pro-inflammatoire.

L'inflammation est fréquente dans presque toutes les maladies qui impliquent des lésions d'origine microbienne, chimique ou physique des tissus vivants. Le résultat favorable de ce processus est l'isolement et la destruction de l'agent nocif, puis la réparation de la lésion inflammatoire afin que l'état normal des tissus soit complètement rétabli. L'inflammation aigüe peut être définie comme la réponse de la microcirculation suite à une lésion ; les symptômes cardinaux sont la chaleur, la rougeur, la douleur, la tuméfaction et la perte fonctionnelle. Les modifications circulatoires comprennent une vasodilatation et une migration des globules blancs et des leucocytes vers la lésion. Lorsque cependant, l'action de l'agent persiste, l'inflammation peut devenir chronique et les modifications circulatoires caractéristiques de l'inflammation aigüe sont dans ce cas remplacées par des lésions chroniques spécifiques, dont la destruction des tissus et la formation de tissus fibreux. Dans les deux cas, les effets indésirables de l'inflammation sont conséquents, et c'est la raison pour laquelle ne pas exacerber cet état inflammatoire tout en prévenant ou en traitant une infection microbienne est intéressant.

L'association des produits Betapur™ et Dermapur® apparait être particulièrement efficace. Les données découlant des études réalisées montrent en particulier que les deux produits Betapur™ et Dermapur®, associés dans un produit de combinaison permettent de stimuler l'expression de la cathélicidine cCath de façon synergique. En effet, chez le chien atopique, il a été observé que le produit Dermapur® (connu chez l'Homme pour stimuler la synthèse cellulaire des cathélicidines) à une concentration de 0,2% en poids du poids total du produit, diminuait l'expression de la cathélicidine cCath chez les chiens atopiques. En revanche, de façon surprenante, il a été observé que le produit Betapur™ à une concentration de 0,2% en poids du poids total du produit augmentait l'expression de la cathélicidine cCath.

De façon plus surprenante encore, il a été observé que la composition associant une combinaison de Betapur™ à une concentration de 0,1% en poids du poids total du produit (concentration donc deux fois inférieure à la concentration du produit Betapur™ testé seul) avec du Dermapur® à une concentration de 0,1% en poids du poids total du produit (concentration également deux fois inférieure à la concentration du produit Dermapur® testé seul) permettait d'obtenir un effet sur la stimulation de la cathélicidine cCath plus grand que la somme des effets attendus lorsque ces produits sont pris indépendamment. Ainsi, l'association des produits Betapur™ et Dermapur®, et donc l'association d'extraits de boldo *Peumus boldus* et d'extraits de reine des prés *Spiraea ulmaria,* permet de créer un effet que chacun d'entre eux ne peut pas obtenir en agissant isolément.

L'invention a également pour objet une composition pour son utilisation dans la prévention, le traitement ou l'aide au contrôle des infections microbiennes chez les animaux.

L'invention a également pour objet une composition pour son utilisation comme stimulateur de la production de peptides antimicrobiens.

L'invention a également pour objet l'utilisation non-thérapeutique d'une telle composition en tant que désinfectant cutané.

Elle a pour autre objet l'utilisation non-thérapeutique d'une telle composition en tant que nettoyant auriculaire.

Elle a pour objet additionnel un procédé non-thérapeutique de stimulation chez un animal des cellules productrices de peptides antimicrobiens tels que les cathélicidines et/ou les béta-défensines, caractérisé en ce que l'on applique sur l'animal une composition selon l'invention.

La présente invention concerne en outre une méthode permettant de réduire la prolifération microbienne par une application topique de la composition selon l'invention, ladite application topique signifiant une application locale sur la peau ou sur une muqueuse.

Enfin, l'invention a pour dernier objet un procédé de fabrication d'une composition selon l'invention, caractérisé en ce que l'on associe au sein d'une même formulation une composition contenant des extraits de boldo *Peumus Boldus* et une composition contenant des extraits de reine des prés *Spiraea ulmaria.*

L'invention sera mieux comprise à la lecture de la description non limitative qui suit, rédigée au regard des figures annexées, dans lesquelles :

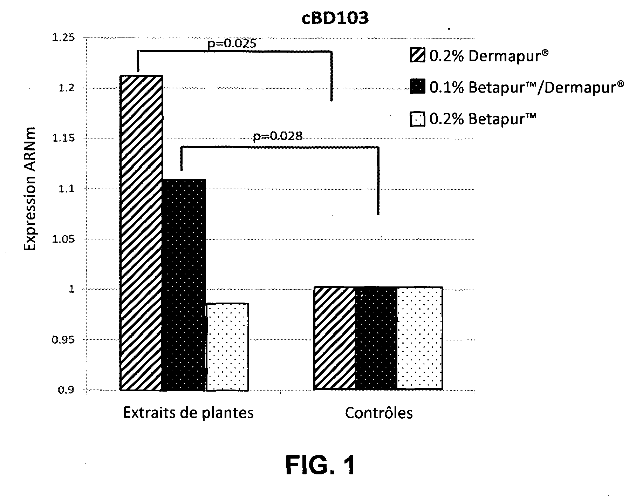

- La figure 1 représente les résultats d'études des effets des produits Betapur™ et/ou Dermapur® sur les niveaux d'expression de l'ARNm de la béta-défensine cBD103. Plus particulièrement, les produits testés sont :
   ∘ Betapur™ à une concentration de 0,2% (ce qui signifie que l'extrait de boldo *Peumus Boldus* est compris entre 0,01% et 0,02% en poids du poids total du produit);
   ∘ Dermapur® à une concentration de 0,2%(ce qui équivaut à 0,0038% en poids du poids total du produit en extrait de reine des prés *Spiraea ulmaria),* et
   ∘ un mélange Betapur™ 0,1%/Dermapur® 0,1%( ce qui signifie que l'extrait de boldo *Peumus Boldus* est compris entre 0,005% et 0,01% en poids du poids total du produit, et que l'extrait de reine des prés *Spiraea ulmaria* équivaut à 0,0019% en poids du poids total du produit)
- La figure 2 représente les résultats d'études des effets des mêmes produits Betapur™ et/ou Dermapur®, aux mêmes concentrations qu'à la figure 1 sur les niveaux d'expression de l'ARNm de la cathélicidine cCath.
- La figure 3 représente les résultats d'études des effets des mêmes produits Betapur™ et/ou Dermapur®, sur la sécrétion de l'interleukine IL-8.
- La figure 4 représente les résultats d'études des effets des mêmes produits Betapur™ et/ou Dermapur®, sur la sécrétion de TNF-alpha.

La composition selon l'invention comprend, dans un milieu physiologiquement acceptable, des extraits de boldo *Peumus boldus* et des extraits de reine des prés *Spiraea ulmaria.*

Par milieu physiologiquement acceptable, on entend selon la présente invention, sans être limitatif, une solution aqueuse ou hydro alcoolique, une émulsion eau-dans-huile, une émulsion huile-dans-eau, une microémulsion, un gel aqueux, un gel anhydre, un sérum, une dispersion de vésicules, une poudre. Physiologiquement acceptable signifie que les compositions conviennent particulièrement à une utilisation topique ou transdermique, en contact avec les muqueuses, les phanères (ongles, poils), les poils et la peau de mammifères et plus particulièrement de chiens et de chats, des compositions pouvant être ingérées ou injectées dans la peau, sans risque de toxicité, d'incompatibilité, d'instabilité, de réponse allergique, et autres.

Ce milieu physiologiquement acceptable forme ce que l'on appelle classiquement l'excipient de la composition.

Pour l'utilisation selon l'invention, la quantité efficace des extraits de boldo *Peumus boldus* et/ou des extraits de reine des prés *Spiraea ulmaria,* c'est-à-dire leur dosage, dépend de divers facteurs, comme l'âge, l'état de la peau de l'animal, etc. Une quantité efficace signifie une quantité non toxique suffisante pour obtenir l'effet désiré.

Le boldo, *Peumus boldus* est un arbuste originaire des zones arides du Chili. Il a également été acclimaté dans le sud de la France et les pays méditerranéens. La surface des feuilles, rugueuse, est couverte de petites vésicules, remplies d'huiles essentielles. Le boldo est une espèce d'arbres de la famille des Monimiaceae.

Les feuilles de boldo contiennent un alcaloïde : la boldine. Elles sont connues pour avoir notamment des propriétés diurétiques, hépatotoniques et sédatives.

La Reine-des-prés, *Filipendula ulmaria* (aussi appelée *Spiraea ulmaria)* est une plante herbacée vivace de la famille des Rosacées, originaire de l'Europe. Les feuilles et les fleurs sont connues pour avoir notamment des propriétés diurétiques, fébrifuges, antispasmodiques et antirhumatismales.

Selon l'invention, la concentration en extraits de boldo de la composition est comprise entre 0,00005% à 50%, préférentiellement entre 0,00005% et 10%, préférentiellement entre 0,00005% à 1%, préférentiellement entre 0,0005% et 0,1% et encore plus préférentiellement entre 0,005% et 0,01% poids total de la composition.

La concentration en extraits de reine des prés de la composition est comprise entre de 0,00001% et 50%, préférentiellement entre 0,00001% et 10%, préférentiellement entre 0,00001% et 0,2%, préférentiellement entre 0,0001% et 0,02% et encore plus préférentiellement entre 0,001 et 0,01% en poids du poids total de la composition.

L'Homme du Métier est capable d'ajuster la quantité d'extraits en fonction de l'effet désiré.

De préférence encore, la concentration en extraits de boldo contient avantageusement entre 0,005% et 0,01% en poids du poids total de la composition, et la concentration en extrait de reine des prés contient avantageusement entre 0,001% et 0,01% en poids du poids total de la composition.

Cependant, la quantité précise de chaque extrait de plante (ou produit comprenant un extrait de plante) est déterminée par l'utilisation finale de la composition selon l'invention. Ce choix dépend notamment d'un ou plusieurs paramètres tels que le type de composant dans lequel ces extraits vont être ajoutés, la forme galénique du produit fini, l'espèce, l'âge, le poids corporel, la santé générale, le sexe, le type de maladie ou d'affection à traiter, de l'animal à qui s'adresse la composition selon l'invention. Par conséquent, les quantités des ingrédients ou composants additionnels peuvent varier considérablement.

De façon avantageuse, les extraits de boldo *Peumus boldus* contenus dans la composition selon l'invention sont issus d'une composition, commercialisée sous le nom Betapur®, comprenant :
- entre 10% et 25% en poids du poids total de la composition de butylène glycol ;
- entre 5% et 10% en poids du poids total de la composition d'extraits de boldo ;
- entre 5% et 10% en poids du poids total de la composition de pentylène glycol ;
- entre 0,1% et 1% en poids du poids total de la composition de gomme xanthane ; et
- plus de 50% en poids du poids total de la composition d'eau ;
et les extraits de reine des prés *Spiraea ulmaria* contenus dans la composition selon l'invention sont issus d'une composition, commercialisée sous le nom Dermapur®, comprenant :
- entre 30% et 70% en poids du poids total de la composition de butylène glycol ;
- entre 1% et 5% en poids du poids total de la composition d'extraits de reine des prés *Spiraea ulmaria ;*
- plus de 25% en poids du poids total de la composition d'eau.

Le produit Betapur™, commercialisé par la société BASF, comprend de l'extrait de boldo, *Peumus boldus.* La composition de ce produit Betapur™ est détaillée dans le tableau 1 ci-dessous :

**Tableau 1 : Composition du produit Betapur™ :**

| Ingrédients | Nom INCI | Pourcentage |
|---|---|---|
| Eau déminéralisée | Water | >50,00% |
| Butylène glycol | Butylene glycol | 10-25% |
| Boldo | *Peumus Boldus* Leaf Extract | 5-10% |
| Hydrolite-5 | Pentylene Glycol | 5-10% |
| Gomme Xanthane | Xanthan Gum | 0,1-1% |

Le produit Dermapur®, commercialisé par la société Silab, comprend de l'extrait de reine des prés, *Spiraea ulmaria* (ou Meadowsweet en anglais). La composition de ce produit Dermapur® est détaillée dans le tableau 2 ci-dessous :

**Tableau 2 : Composition du produit Dermapur® :**

| Ingrédients | Pourcentage | N° CAS | N° EINECS |
|---|---|---|---|
| Butylène glycol | 50,00% | 107-88-0 | 203-529-7 |
| Eau | 48,10% | 7732-18-5 | 231-791-2 |
| Extrait de *Spiraea Ulmaria* | 1,90% | 84775-57-5 | 283-886-3 |

La composition selon l'invention comprend un rapport pondéral entre les extraits de boldo et les extraits de reine des prés compris entre 99 pour 1 et 1 pour 99, préférentiellement entre 50 pour 1 et 1 pour 50, encore plus préférentiellement entre 10 pour 1 et 1 pour 10, encore plus préférentiellement entre 10 pour 1 et 2 pour 1, de manière encore plus préférée entre 6 pour 1 et 3 pour 1.

Selon un mode particulier de réalisation de l'invention, la composition comprend comme uniques extraits de plantes stimulant la synthèse cellulaire de peptides antimicrobiens :
- des extraits de boldo, *Peumus boldus,* et
- des extraits de reine des prés *Spiraea ulmaria.*

Comme il en découle des études réalisées par la Demanderesse, une composition comprenant au moins des extraits de boldo *Peumus boldus* et/ou des extraits de reine des prés *Spiraea ulmaria* est particulièrement adaptée pour une utilisation dans la prévention ou le traitement des infections microbiennes De même, une telle composition est adaptée à une utilisation dans l'aide au contrôle des infections microbiennes chez les animaux.

De façon avantageuse, les compositions selon l'invention sont adaptées à une utilisation dans la prévention, le traitement ou dans l'aide au contrôle des infections microbiennes, notamment les infections par *Staphylococcus pseudintermedius* et/ou *Malassezia pachydermatis*.

Selon l'invention, les compositions sont utilisables comme stimulateurs de la production de peptides antimicrobiens, notamment les cathélicidines et/ou les béta-défensines.

De façon surprenante, la composition selon l'invention ne stimule pas la production de cytokines pro-inflammatoires.

De préférence, l'animal à qui l'on administre la composition selon l'invention présente une altération de la barrière cutanée.

Les lésions cutanées peuvent notamment être causées par des dermatites (dermatites à *Malassezia,* dermatite atopique), des pyodermites, des otites chroniques ou des infections fongiques ou bactériennes.

Selon un autre aspect de l'invention, la composition objet de l'invention est administrée quotidiennement pour une période courte d'au moins 3, 5, 7, 15 ou 21 jours, ou pour une période plus longue d'au moins 1, 2, 3, 4, 5 ou 6 mois avec des fréquences d'administration variant de 1 à 3 fois/semaine.

Les différentes formes galéniques pouvant contenir les extraits de boldo et les extraits de reine des prés selon l'invention se présentent sous toute forme à savoir crèmes, lotions, onguents, laits, gels, émulsions, dispersions, solutions, suspensions, shampooings, patchs transdermiques, ou poudres, sprays, pommades, liniments, formulations pulvérisables, brossables, savons. Les solutions ou suspensions qui doivent être appliquées localement sont avantageusement présentées en spot-on, c'est-à-dire qu'elles sont appliquées par dépôt en un ou deux points localisés, sur la peau de l'animal. On peut également envisager une composition sous la forme d'une mousse ou encore sous forme de composition pour aérosol comprenant également un agent propulseur sous pression. Les compositions selon l'invention peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectant, des agents de polissage tels que la silice, divers ingrédients actifs comme les fluorures, en particulier le fluorure de sodium, et éventuellement des agents édulcorants comme le saccharinate de sodium. Avantageusement, la composition selon l'invention comprend des agents tensioactifs tels qu'émulsifiants et humectants, choisis parmi les dérivés anioniques, cationiques, amphotères ou non-ioniques, les stabilisants tels que des antioxydants et les colorants.

De façon avantageuse, les compositions selon l'invention peuvent se présenter sous forme de gels, de lotions, d'émulsions huile dans l'eau ou eau dans l'huile, de dispersions, de laits, de crèmes, d'onguents, de mousses, de shampoings, de solutions micellaires ou sous toute autre forme galénique appropriée à une application topique. Ces différentes formes peuvent être préparées par l'Homme du Métier par les techniques couramment en usage dans l'industrie des cosmétiques et pharmaceutique.

Les compositions selon l'invention sont avantageusement conditionnés dans des flacons, bouillottes, tubes, spray, aérosols, nettoyants auriculaires (ear cleanser), pipette , lingettes, sachets ou dans tout autre conditionnement approprié à une application topique.

Préférentiellement, la composition est appliquée à l'animal sous la forme d'un shampoing, d'une lotion, d'une crème, d'une pommade, d'un gel ou d'une solution ou d'une suspension en spot-on.

Par application topique, on entend une application qui est destinée à agir à l'endroit où elle est appliquée: peau, muqueuse, phanères. L'utilisation topique ou l'application topique selon l'invention peut avantageusement viser les peaux fatiguées (ayant par exemple perdu de leur souplesse), les peaux abîmées et/ou irritées (suite aux agressions des éléments tels que le froids, le soleil) ou encore les peaux blessées par des griffures, des plaies, des infections (bactériennes ou fongiques par exemple). Ainsi l'application topique de la composition selon l'invention peut se faire, selon les cas et l'état des animaux concernés, sur des animaux sains, en entretien comme par exemple la désinfection cutanée et en particulier le nettoyage auriculaire, ou sur des animaux blessés et/ou malades en traitement et en prévention en particulier des infections par *Staphylococcus pseudintermedius* et/ou *Malassezia pachydermatis.*

Des améliorations de l'apparence et de l'état général de la peau des muqueuses et des phanères peuvent être obtenues par application topique de manière régulière comme par exemple de façon quotidienne. Le praticien appréciera le traitement topique cosmétique qui comprendra une composition contenant les extraits de boldo *Peumus boldus* et/ou les extraits de reine des prés *Spiraea ulmaria*, ce traitement pouvant être réalisé par exemple en appliquant topiquement la composition décrite dans la présente invention, selon une technique habituellement utilisée pour appliquer une telle composition.

L'application topique peut être cosmétique et/ou dermopharmaceutique et/ou pharmaceutique.

De préférence, la composition selon l'invention est appliquée sur la peau ou le pelage d'un animal, qui est préférentiellement un chat ou un chien.

De façon avantageuse, la composition selon l'invention peut également comprendre un ou plusieurs ingrédients additionnels bien connus de l'Homme du Métier tels que notamment, les stabilisants, les agents diluants, les parfums, les charges, les sels, les agents nettoyants qui peuvent être des agents surfactants et/ou des tensioactifs et/ou des agents émulsifiants, les polymères (incluant les matériaux obtenus par polymérisation d'un type de monomère ou obtenus à partir de deux types de monomères (copolymères) ou à partir de plusieurs types de monomères), les agents antimicrobiens, les agents moussants, les synergistes de mousse, les agents antimousses, les agents de foisonnement, les agents de protection de la peau, les agents démêlants, les agents filmogènes, les agents de gélification, les conditionneurs capillaires, les stabilisateurs d'émulsion, les émollients, les agents tampons, les agents antistatiques, les agents kératolytiques, les agents stabilisateurs, les agents de chélation, les antioxydants, les agents antiséborrhéiques, les agents de réduction, les agents abrasifs, les agents absorbants, les agents antiagglomérants, les agents anticorrosions, les agents antipelliculaires, les agents antiperspirants, les agents déodorants, les agents astringents, les plastifiants, les propulseurs, les liants, les dénaturants, les agents de restauration lipidique, les agents masquants, les agents colorants, les agents nacrants, les opacifiants, les agents apaisants, les hydratants, les agents lissants, les agents rafraîchissants, les agents d'oxydation, les solvants, les tonifiants, les absorbants UV, les filtres UV, les agents de contrôle de la viscosité.

Les antioxydants peuvent avantageusement être choisis parmi le tocophérol et ses esters, tels que l'acétate de tocophérol ; le BHT et le BHA.

Les agents antimicrobiens susceptibles d'être utilisés dans la composition selon l'invention sont préférentiellement choisis parmi le 2,4,4 '-trichloro-2 '-hydroxy diphenyl ether (ou triclosan), le 3,4,4 '-trichlorobanilide, le phenoxyethanol, le phenoxypropanol, le phenoxyisopropanol, l'hexamidine isethionate, le metronidazole et ses sels, le miconazole et ses sels, l'itraconazole, le terconazole, l'econazole, le ketoconazole, le saperconazole, le fluconazole, le clotrimazole, le butoconazole, 1' oxiconazole, le sulfaconazole, le sulconazole, la terbinafine, le ciclopiroxe, le ciclopiroxo lamine, l'acide undecylenique et ses sels, le peroxyde de benzoyle, l'acide 3-hydroxy benzoïque, l'acide 4-hydroxy benzoïque, l'acide phytique, l'acide N-acétyl L-cystéine, l'acide lipoïque, l'acide azelaïque et ses sels, l'acide arachidonique, le resorcinol, le 2,4,4' -trichloro-2' - hydroxy diphenyl ether, le 3,4,4' -trichlorocarbanalide, l'octopirox, l'octoxyglycérine, l'octanoylglycine, le caprylyl glycol, l'acide 10-hydroxy-2-decanoïque, le pidolate de cuivre, l'acide salicylique, la salicylate de zinc, l'iodopropynyl butylcarbamate, le famesol, les phytosphingosines et leurs mélanges.

Comme agent surfactant peuvent être cités les tensioactifs anioniques, cationiques, zwitterioniques (par ex les dérivés sulfonate, sulfate, carboxylate, phosphonate, phosphate) amphotères, non chargé (par ex les macrogols) et un mélange de ceux-ci.

Les agents stabilisateurs comprennent les substances qui ont tendance à augmenter la durée de conservation de la composition tels que les conservateurs, les émulsifiants, les épaississants, les gaz d'emballage, les gélifiants, les hydrotropes, les humectants, les séquestrants, ou les stabilisants.

Parmi les agents diluants on pourra citer des sels minéraux insolubles ou peu solubles dans l'eau tels que des carbonates de métal alcalinoterreux de magnésium ou de zinc, des titanates de métal alcalin ou de métal alcalino-terreux, du dioxyde de titane, des oxydes métalliques comme des oxydes de fer, de l'oxyde de zinc, de l'oxyde d'étain, des sulfates de métal alcalino-terreux comme le sulfate de calcium ou le sulfate de baryum, des phosphates mono-, di-ou tri-basiques comme l'hydrogéno phosphate de calcium, le phosphate tricalcique ou le phosphate de magnésium.

La composition selon l'invention est destinée aux animaux, et préférentiellement à un animal domestique, c'est-à-dire un animal issu de la domestication et de l'élevage. Cela comprend le bétail, les animaux de basse-cour, les animaux aquatiques ou encore les animaux de compagnie. De préférence, l'animal domestique est un animal de compagnie choisi parmi les mammifères (canidés, félidés, équidés, lagomorphes, rongeurs). De façon avantageuse, la composition selon l'invention est destinée aux chiens et aux chats, cependant, le terme animal de compagnie inclut également les nouveaux animaux de compagnie (NAC) tels que par exemple le furet, les rongeurs (par exemple le hamster), les lagomorphes (tels que par exemple, le lapin), le cochon nain.

Dans un aspect de l'invention, la composition selon l'invention est destinée aux chiens. La composition et les méthodes selon l'invention sont plus particulièrement destinées aux chiens ayant une altération de la barrière cutanée. C'est le cas notamment de la dermatite atopique. Il s'agit d'une maladie avec une prédisposition raciale. Parmi ces races de chien peuvent être citées, de manière non limitative: Boston Terrier, Cairn Terrier, Dalmatien, English Bulldog, English Setter, Golden Retriever, Golden Retriever Irish Setter, Shar-pei, Labrador Retriever, Lhasa Apso, Schnauzer Nain, Carlin, Scottish Terrier, West Highland White Terrier, Fox Terrier à poil dur, Boxer, Berger Allemand, Caniche, Fox Terrier, Cocker Américain, Bull Terrier Chow-Chow, Berger des Pyrénées, Chien de Berger Belge, Shih Tzu, Chihuahua, Yorkshire Terrier, Gordon Setter.

Des chiens qui ne sont pas de race pure mais qui possèdent des caractéristiques proches desdites races peuvent également être traités en utilisant la composition selon la présente invention.

Dans un mode de réalisation de l'invention, la composition selon l'invention peut être administrée en combinaison avec des médicaments usuellement prescrits aux animaux souffrant d'infections microbiennes ou d'altérations de la barrière cutanée ou à tout autre agent pharmaceutique destiné à lutter contre les parasites externes tels que les puces, tiques, poux qui sont susceptibles de causer des altérations de la peau en conduisant par exemple l'animal à se gratter. On citera en particulier les agents insecticides tels que le fipronil, l'imidaclopride, l'amitraz, la permethrine, le méthoprène, le pyriproxyfène et tout autre principe actif capable de prévenir ou de traiter les infestations par les ectoparasites.

La composition peut notamment aussi comprendre des antibiotiques systémiques et topiques, tels que les aminosides, comme la gentamycine, la streptomycine, l'amikacine, la néomycine, la tobramycine, la kanamycine ; les bêta-lactamines tels que les pénicillines (l'amoxicilline avec ou sans acide clavulanique par exemple), la pénicilline G, l'ampicilline, mais aussi les céphalosporines avec la céfalexine, la céfovécine, la cefadroxil, la cefquinome, la cefpodoxime, ou le ceftiofur. Les antibiotiques systémiques et topiques comprennent aussi les cyclines comme les tétracyclines, les oxytétracyclines, la doxycycline, mais encore les macrolides (avec l'érythromycine, l'azythromycine, la spiramycine), les lincosamides comme la clindamycine, la lincomycine, et aussi les fluoroquinolones : l'enrofloxacine la marbofloxacine, la pradofloxacine , la difloxacine, l'orbifloxacine. Sont aussi considérés comme tels les sulfamides avec la sulfaméthoxypyridazine, le sulfaméthoxazole (avec ou sans triméthoprime), la sulfadiazine , mais aussi des rifamycines comme la rifampicine, des polypeptides avec notamment la colistine, ou encore d'autres antibiotiques comme le métronidazole, ou l'acide fusidique.

Elle peut aussi être administrée en combinaison avec des antifongiques systémiques et topiques, et notamment les polyènes, comme l'amphotéricine B, la nystatine, et la griséofulvine, ainsi que les azolés tels que le fluconazole, l'itraconazole, le posaconazole, le miconazole, l'enilconazole, le kétoconazole, le fluconazole, le climbazole, ou encore les allylamines avec notamment la terbinafine.

La composition selon l'invention peut aussi contenir des antiseptiques, comme par exemple les produits chlorés tels que l'hypochlorite de sodium (et le soluté de Dakin),les halogénés avec des produits iodés (ce qui comprend l'iode et ses dérivés comme l'alcool iodé ou la teinture d'iode), des iodophores comme le polyvidone iodée, mais aussi des biguanides (comme la chlorhexidine), des alcools, des ammoniums quaternaires : chlorure de benzalkonium, bromure de cetrimemium, ou encore des oxydants, comme le peroxyde d'hydrogène (c'est-à-dire l'eau oxygénée). Les antiseptiques comprennent aussi les carbanilides tels que le triclocarban, et le triclosan, mais aussi d'autres molécules, comme le peroxyde de benzoyle, l'éthyl lactate, la piroctone olamine, l'acide benzoïque, l'acide acétique, l'acide borique, l'hexétédine, l'EDTA avec ou sans tris, le PCMX.

De la même façon, la composition peut aussi comprendre des immunomodulateurs, notamment des corticostéroïdes (prednisolone, prednisone, dexaméthasone, bétaméthasone, hydrocortisone, hydrocortisone acéponate, mométasone, triamcinolone, prednicarbate, clobétasol, fluticasone), des inhibiteurs de calcineurine (ciclosporine, tacrolimus), des interférons, des acides gras essentiels, des antihistaminiques. Elle peut aussi être combinée avec des agents restructurants et réhydratants avec des céramides, des acide gras, du cholestérol ou de l'urée, mais encore avec des sébomodulateurs tels que la vitamine B6,ou le zinc.

Cette liste non exhaustive des différents médicaments usuellement prescrits aux animaux souffrant d'infections microbiennes ou d'altérations de la barrière cutanée est bien connue de l'Homme du Métier.

La composition selon l'invention peut également comprendre des substances autres que les extraits de plantes de boldo *Peumus boldus* et de reine des prés *Spiraea ulmaria* détaillées ci-dessus, notamment d'autres extraits de plantes, des matières premières alimentaires (végétales, animales, minérales), des acides aminés, des protéines, des huiles essentielles, des matières grasses (acides gras en particulier), des minéraux, des vitamines ou des substances actives médicamenteuses.

Comme cela est détaillé dans les essais présentés ci-dessous, selon l'invention, la composition est avantageusement utilisée pour le traitement, la prévention ou l'aide au contrôle des infections microbiennes. Dans le cadre de la présente invention, on entend par « aide au contrôle des infections microbiennes », un moyen d'apporter un support à la barrière épidermique et/ou aux défenses immunitaires microbiennes innées lors d'infections microbiennes chez des animaux.

Selon une variante, l'invention consiste en une composition comprenant dans un milieu physiologiquement acceptable des extraits de boldo *Peumus boldus* et/ou en une composition comprenant dans un milieu physiologiquement acceptable des extraits de reine des prés *Spiraea ulmaria.* Lesdites composition sont utilisées en association pour leur utilisation dans la prévention, le traitement ou l'aide au contrôle des infections bactériennes chez les animaux. Ces compositions selon l'invention peuvent être appliquées de manière topique sur la peau ou le pelage d'un animal, préférentiellement un chat ou un chien. La concentration en extraits de boldo est comprise entre 0,00005% à 50%. La concentration en extraits de reine des prés est comprise entre de 0,00001% et 50%. Cette variante de l'invention a aussi pour objet, l'utilisation de chacune de ces compositions, en association comme stimulateur de la production de peptides antimicrobiens, tels que les cathélicidines et/ou les béta-défensines, sans pour autant stimuler la production de cytokines pro-inflammatoires.

Dans une variante de l'invention, la composition peut être administrée de manière quotidienne et ce, tout au long de la vie de l'animal. Dans une autre variante, la composition peut également être administrée de manière régulière 1, 2, 3 fois par jour ou tous les 2, 3, 4 jours, 1, 2, 3 fois par semaine ou 1, 2, 3, 4 fois par mois.

Dans une autre variante, la composition peut également être administrée sous forme de cures ou cycles de traitement. C'est-à-dire que l'administration de la composition, quotidienne ou non, est faite sur une période de temps définie, comprise entre 1 semaine et un an, plus particulièrement pendant 1, 2, 3, 4, 5 ou 6 mois. Dans une variante de l'invention, la composition selon l'invention est administrée au moins 2, 3, 4, 5, 6 ou plusieurs fois à l'animal. Dans un mode de réalisation, la composition est administrée sous forme de cure de 1, 2, 3, 4, 5, 6 ou plusieurs mois, 1, 2, 3 fois par année. Dans une variante particulière de l'invention, la composition est administrée quotidiennement, pour une période comprise entre 1 et 6 mois, sous forme de cure renouvelable une à deux fois par an.

Dans un autre variante particulière, la composition est administrée quotidiennement pour une période d'au moins 1, 2, 3, 4, 5 ou 6 mois.

La composition selon l'invention est administrée dans un dose et à une fréquence qui peuvent être choisies et ajustées par l'Homme du Métier.

Dans un aspect de l'invention, la composition est utilisée à des fins non-thérapeutiques, par exemple pour améliorer l'état général de l'animal. En effet, la composition selon l'invention peut avoir un effet énergisant, un effet sur la diminution du vieillissement cellulaire et par voie de conséquence réduire l'incidence de maladies et favoriser le rétablissement de l'animal après une maladie ou un accident, ou encore sur l'augmentation de la vitalité. Ces bienfaits seront visibles par une amélioration de l'aspect esthétique de l'animal (pelage brillant, meilleure posture), de son tonus, par l'amélioration de son comportement (interaction avec son maître, sommeil, appétit, plaisir au jeu), l'amélioration de l'état général évitera également l'installation de maladies opportunistes.

### Exemple 1 : Evaluation de l'effet in vitro d'extraits de plantes sur la production de peptides antimicrobiens et des marqueurs de l'inflammation dans les kératinocytes canins.

Comme indiqué ci-dessus, les peptides antimicrobiens (PAMs) sont de petits peptides produits par les cellules épithéliales et immunitaires. Ils jouent un rôle important dans la défense immunitaire microbienne innée. Les béta-défensines (BDs) et les cathélicidines (Cath) sont les PAMs les plus étudiés.

L'objectif de la présente étude est double. Il est de :
- tester si les extraits de plantes sont capables de stimuler la production de BD103 canine et/ou de Cath canine sur des kératinocytes canins en culture primaire ;
- évaluer leur réponse pro-inflammatoire (production de cytokines) dans le surnageant de culture cellulaire.

Cette étude a été réalisée en deux phases. La phase I a été réalisée sur 10 chiens de race Beagles, 5 normaux et 5 atopiques. La phase II a été réalisée sur 14 chiens atopiques de race Beagles.

Cette étude a été réalisée sur 10 chiens de race Beagles, 5 normaux et 5 atopiques. Trois biopsies de 8 mm ont été effectuées sur chaque chien. Les kératinocytes ont été collectés en utilisant des techniques de prélèvement et de mise en culture standards. Ils ont été répartis dans 24 plaques de 48 puits de culture ainsi.

Du milieu de culture adapté à la culture de kératinocytes primaires (milieu Cn-T09™, commercialisé par la société Cellntech) de chien a été utilisé. La toxine du choléra a été ajoutée à ce milieu. Le milieu a été changé tous les 3 jours jusqu'à obtenir 70% de confluence. Les cellules ont ensuite été cultivées dans un milieu appauvri en facteurs de croissance cellulaire pendant 24 heures, puis diverses concentrations des d'extraits de boldo et de reine des prés ont été ajoutées pendant 24 heures.

L'extrait de boldo a été testé à différentes concentrations (2%, 1,5%, 1%, 0,4% et 0,2%). L'extrait de boldo correspond au produit Betapur™ commercialisé par la société BASF qui comprend de l'extrait de boldo, *Peumus boldus.* La composition de ce produit Betapur™ est détaillée dans le tableau 1 ci-dessus.

L'extrait de reine des prés est testé à différentes concentrations (1%, 0,4% et 0,2%). L'extrait B correspond au produit Dermapur® commercialisé par la société Silab qui comprend de l'extrait de reine des prés, *Spiraea ulmaria* (ou Meadowsweet en anglais). La composition de ce produit Dermapur® est détaillée dans le tableau 2 ci-dessus.

Toutes les concentrations ont été testées en double. Après récupération du surnageant, les cellules ont été lysées et l'ARNm a été extrait pour une analyse moléculaire. Le test de normalité Kolmogorov-Smirnov a été utilisé (alpha = 0,05). Les valeurs moyennes et les intervalles de confiance à 95% ont été calculés pour tous les résultats. Les différences entre chaque extrait et son contrôle ont été comparées en utilisant le test t apparié. Les valeurs de p ≤ 0,05 ont été considérées comme significatives.

### Résultats concernant la phase I :

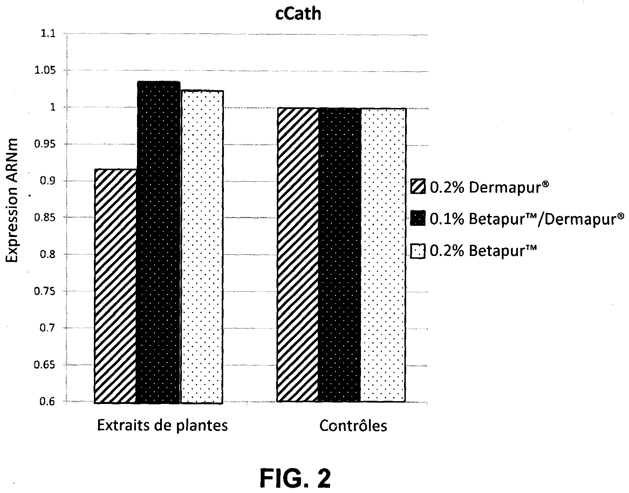

Les études ont permis de mettre en évidence que :
(i) dans les kératinocytes issus de chiens normaux (sains), aucun des produits Betapur™ ou Dermapur®, pris seuls ou en association, n'augmente de façon significative l'expression de la béta-défensine cBD103 et de la cathélicidine cCath.
(ii) dans les kératinocytes issus de chiens normaux (sains), aucun des produits Betapur™ ou Dermapur® n'a d'effet significatif sur la production de TNF-α, cytokine impliquée dans l'inflammation et notamment au niveau de la peau.
(iii) dans les kératinocytes issus de chiens normaux (sains), le produit Betapur™ n'a pas d'effet significatif sur la production d'Interleukine-8 (IL-8), chimiokine recrutant notamment les polynucléaires neutrophiles. En revanche, le produit Dermapur®, à une concentration de 1% augmente la production de l'Interleukine-8 (IL-8).
(iv) Comme cela est illustré à la figure 1, dans les kératinocytes issus de chiens atopiques, il a été observé qu'à la fois :
   o le produit Dermapur® à une concentration de 0,2%, et
   o la composition comprenant une combinaison 0,1% de Dermapur® associé à 0,1% de Betapur™;
   augmentaient de façon significative l'expression de la béta-défensine cBD103 (p=0,025 et p = 0,028 respectivement). En revanche, de façon particulièrement surprenante, le produit Betapur™ à une concentration de 0,2%, connu chez l'Homme pour stimuler l'expression de la béta-défensine humaine hBD-3 n'augmente pas l'expression de la béta-défensine cBD103 chez les chiens atopiques.
(v) Comme cela est illustré à la figure 2, dans les kératinocytes issus de chiens atopiques, il a été observé de façon particulièrement surprenante que le produit Dermapur®, connu chez l'Homme pour stimuler la synthèse cellulaire des cathélicidines, diminuait l'expression de la cathélicidine cCath (p=0,0615) chez les chiens atopiques. En revanche, il a été observé qu'aussi bien le produit Betapur® que la composition comprenant une combinaison de 0,1% de Dermapur® associé à 0,1% de Betapur™ augmentent l'expression de la cathélicidine cCath.

### Résultats concernant la phase II :

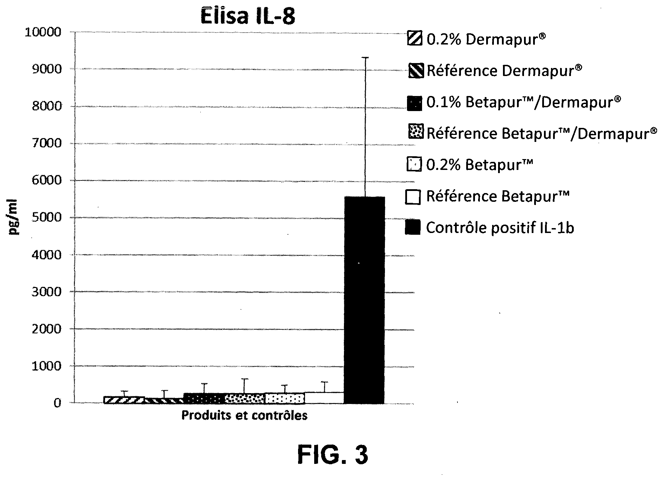

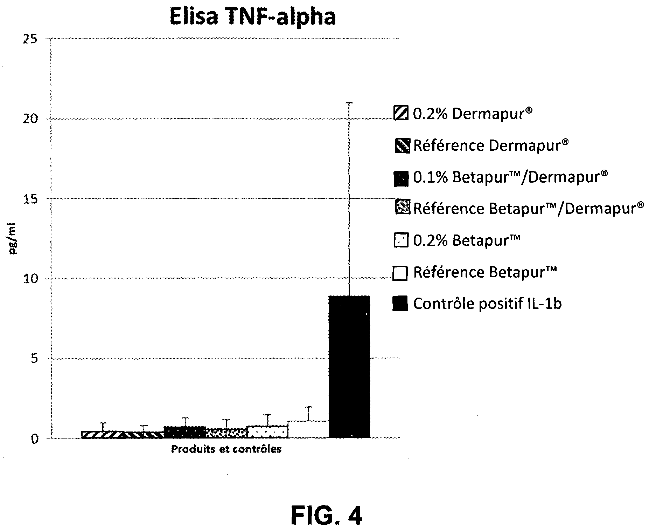

(vi) Enfin, comme cela est illustré aux figures 3 et 4, aucun des produits Dermapur® 0,2%, Betapur™ 0,2% ou la combinaison comprenant 0,1% de Dermapur® associé à 0,1% de Betapur™ n'entraine l'augmentation de la chimiokine IL-8 ou de la cytokine TNF-α. Autrement dit, aucun des deux produits ou la composition comprenant une combinaison des deux produits n'entraîne de réponse pro-inflammatoire.

### Exemple 2 : Shampoing selon l'invention.

Dans un exemple particulièrement adapté à l'aide au contrôle des infections par *Malassezia pachydermatis,* la composition selon l'invention est un shampoing comportant dans un milieu physiologiquement acceptable des extraits de boldo *Peumus boldus* et des extraits de reine des prés *Spiraea ulmaria.*
Pour une utilisation de ce shampoing, la Demanderesse a déterminé la quantité de produit à administrer, qui varie en fonction du poids de l'animal considéré. Cette quantité doit être adaptée à la fois à la taille de l'animal ainsi qu'à la longueur des poils du pelage. Il peut être appliqué selon les critères décrits dans le tableau suivant :

| Poids de l'animal | Volume du produit |
|---|---|
| 4,9 kg | 10 ml x 2 bains |
| 5,0 à 10,9 kg | 15 ml x 2 bains |
| 11,0 à 15,9 kg | 20 ml x 2 bains |
| 16,0 à 20,9 kg | 25 ml x 2 bains |
| 21,0 à 30,9 kg | 30 ml x 2 bains |
| 31,0 à 45,9 kg | 40 ml x 2 bains |
| 46,0 kg | 50 ml x 2 bains |

## Revendications

1. Composition topique pour animaux, **caractérisée en ce qu'**elle comprend dans un milieu physiologiquement acceptable des extraits de boldo *Peumus boldus* et des extraits de reine des prés *Spiraea ulmaria.*

2. Composition selon la revendication 1, **caractérisée en ce qu'**elle est appliquée sur la peau ou le pelage d'un animal.

3. Composition selon la revendication 2, **caractérisée en ce que** l'animal est un chat ou un chien.

4. Composition selon l'une des revendications précédentes, **caractérisée en ce qu'**elle est appliquée à l'animal sous la forme d'un shampoing, d'une lotion, d'une crème, d'une pommade, d'un gel ou d'une solution ou d'une suspension en spot-on.

5. Composition selon l'une des revendications précédentes, **caractérisée en ce que** les extraits de boldo représentent de 0,00005% à 1% en poids du poids total de la composition, et **en ce que** les extraits de reine des prés représentent de 0,00001% à 0,2% en poids du poids total de la composition.

6. Composition selon la revendication 5, **caractérisée en ce que** les extraits de boldo représentent de 0,005% à 0,01% en poids du poids total de la composition, et **en ce que** les extraits de reine des prés représentent de 0,001% à 0,01% en poids du poids total de la composition.

7. Composition selon l'une des revendications précédentes **caractérisée en ce que** le rapport pondéral entre les extraits de boldo et les extraits de reine des prés est compris entre 99 pour 1 et 1 pour 99 et préférentiellement entre 10 pour 1 et 1 pour 10.

8. Composition selon l'une des revendications précédentes, pour son utilisation dans la prévention, le traitement ou l'aide au contrôle des infections microbiennes chez les animaux.

9. Composition pour son utilisation selon la revendication 8, pour son utilisation dans la prévention, le traitement ou dans l'aide au contrôle des infections par *Staphylococcus pseudintermedius* et/ou *Malassezia pachydermatis.*

10. Composition pour son utilisation selon l'une des revendications précédentes, pour son utilisation comme stimulateur de la production de peptides antimicrobiens.

11. Composition pour son utilisation selon la revendication 10, **caractérisée en ce que** les peptides antimicrobiens sont les cathélicidines et/ou les béta-défensines.

12. Composition ou composition pour utilisation selon l'une des revendications précédentes, **caractérisée en ce qu'**elle ne stimule pas la production de cytokines pro-inflammatoires.

13. Composition ou composition pour utilisation selon l'une quelconque des revendications précédentes, **caractérisée** en ce l'animal présente une altération de la barrière cutanée.

14. Composition ou composition pour utilisation selon la revendication 13, **caractérisée en ce que** l'animal est atteint de lésions cutanées causées par des dermatites (dermatites à *Malassezia,* dermatite atopique), des pyodermites, des otites chroniques ou des infections fongiques ou bactériennes.

15. Utilisation non-thérapeutique d'une composition selon l'une quelconque des revendications 1 à 7 en tant que désinfectant cutané.

16. Utilisation non-thérapeutique d'une composition selon l'une quelconque des revendications 1 à 7 en tant que nettoyant auriculaire.

17. Procédé non-thérapeutique de stimulation chez un animal des cellules productrices de peptides antimicrobiens telles que les cathélicidines et/ou les béta-défensines **caractérisé en ce que** l'on applique sur l'animal une composition selon l'une quelconque des revendications 1 à 7.

18. Procédé de fabrication d'une composition selon l'une des revendications 1 à 7, **caractérisé en ce que** l'on associe au sein d'une même formulation une composition contenant des extraits de boldo *Peumus Boldus* et une composition contenant des extraits de reine des prés *Spiraea ulmaria.*

## Patentansprüche

1. Topische Zusammensetzung für Tiere, **dadurch gekennzeichnet, dass** sie in einem physiologisch verträglichen Medium Extrakte von Boldo, *Peumus boldus,* und Extrakte von Mädesüß, *Spiraea ulmaria,* umfasst.

2. Zusammensetzung nach Anspruch 1, **dadurch gekennzeichnet, dass** sie auf der Haut oder dem Fell eines Tieres angewendet wird.

3. Zusammensetzung nach Anspruch 2, **dadurch gekennzeichnet, dass** das Tier eine Katze oder ein Hund ist.

4. Zusammensetzung nach einem der vorstehenden Ansprüche, **dadurch gekennzeichnet, dass** sie in der Form eines Shampoos, einer Lotion, einer Creme, einer Salbe, eines Gels oder einer Lösung oder einer Spot-on-Suspension bei dem Tier angewendet wird.

5. Zusammensetzung nach einem der vorstehenden Ansprüche, **dadurch gekennzeichnet, dass** die Boldo-Extrakte 0,00005 Gew.-% bis 1 Gew.-% des Gesamtgewichts der Zusammensetzung ausmachen, und dadurch, dass die Mädesüß-Extrakte 0,00001 Gew.-% bis 0,2 Gew.-% des Gesamtgewichts der Zusammensetzung ausmachen.

6. Zusammensetzung nach Anspruch 5, **dadurch gekennzeichnet, dass** die Boldo-Extrakte 0,005 Gew.-% bis 0,01 Gew.-% des Gesamtgewichts der Zusammensetzung ausmachen, und dadurch, dass die Mädesüß-Extrakte 0,001 Gew.-% bis 0,01 Gew.-% des Gesamtgewichts der Zusammensetzung ausmachen.

7. Zusammensetzung nach einem der vorstehenden Ansprüche, **dadurch gekennzeichnet, dass** das Gewichtsverhältnis zwischen den Boldo-Extrakten und den Mädesüß-Extrakten im Bereich zwischen 99 zu 1 und 1 zu 99, und vorzugsweise zwischen 10 zu 1 und 1 zu 10 beträgt.

8. Zusammensetzung nach einem der vorstehenden Ansprüche zu deren Verwendung in der Vorbeugung, der Behandlung oder der Hilfe zur Kontrolle der mikrobiellen Infektionen bei Tieren.

9. Zusammensetzung zu deren Verwendung nach Anspruch 8, zu deren Verwendung in der Vorbeugung, der Behandlung oder in der Hilfe zur Kontrolle der Infektionen mit *Staphylococcus pseudintermedius* und/oder *Malassezia pachydermatis.*

10. Zusammensetzung zu deren Verwendung nach einem der vorstehenden Ansprüche, zu deren Verwendung als Stimulator der Produktion von antimikrobiellen Peptiden.

11. Zusammensetzung zu deren Verwendung nach Anspruch 10, **dadurch gekennzeichnet, dass** die antimikrobiellen Peptide Cathelicidine und/oder beta-Defensine sind.

12. Zusammensetzung oder Zusammensetzung zur Verwendung nach einem der vorstehenden Ansprüche, **dadurch gekennzeichnet, dass** sie die Produktion von proinflammatorischen Zytokinen nicht stimuliert.

13. Zusammensetzung oder Zusammensetzung zur Verwendung nach einem der vorstehenden Ansprüche, **dadurch gekennzeichnet, dass** das Tier eine Veränderung der Hautbarriere aufweist.

14. Zusammensetzung oder Zusammensetzung zur Verwendung nach Anspruch 13, **dadurch gekennzeichnet, dass** das Tier an Hautläsionen leidet, die von Dermatiden (*Malassezia-*Dermatiden, atopische Dermatitis), Pyodermien, chronischen Otitiden oder Pilz- oder bakteriellen Infektionen verursacht werden.

15. Nicht therapeutische Verwendung einer Zusammensetzung nach einem der Ansprüche 1 bis 7 als Hautdesinfektionsmittel.

16. Nicht therapeutische Verwendung einer Zusammensetzung nach einem der Ansprüche 1 bis 7 als Ohrreinigungsmittel.

17. Nicht therapeutisches Verfahren zur Stimulation der antimikrobielle Peptide wie etwa Cathelicidine und/oder beta-Defensine produzierenden Zellen bei einem Tier, **dadurch gekennzeichnet, dass** eine Zusammensetzung nach einem der Ansprüche 1 bis 7 bei dem Tier angewendet wird.

18. Verfahren zur Herstellung einer Zusammensetzung nach einem der Ansprüche 1 bis 7, **dadurch gekennzeichnet, dass** in ein und derselben Formulierung eine Zusammensetzung, die Extrakte von Boldo, *Peumus Boldus,* enthält, und eine Zusammensetzung, die Extrakte von Mädesüß, *Spiraea ulmaria,* enthält, zusammengeführt werden.

## Claims

1. Topical composition for animals, **characterised in that** it comprises, in a physiologically acceptable medium, extracts of boldo *Peumus boldus* and extracts of meadowsweet *Spiraea ulmaria.*

2. Composition according to claim 1, **characterised in that** it is applied on the skin or fur of an animal.

3. Composition according to claim 2, **characterised in that** the animal is a cat or a dog.

4. Composition according to one of the preceding claims, **characterised in that** it is applied to the animal in the form of a shampoo, a lotion, a cream, an ointment, a gel or a solution or a suspension by spot-on application.

5. Composition according to one of the preceding claims, **characterised in that** the extracts of boldo represent from 0.00005% to 1% by weight of the total weight of the composition, and **in that** the extracts of meadowsweet represent from 0.00001% to 0.2% by weight of the total weight of the composition.

6. Composition according to claim 5, **characterised in that** the extracts of boldo represent from 0.005% to 0.01% by weight of the total weight of the composition, and **in that** the extracts of meadowsweet represent from 0.001% to 0.01% by weight of the total weight of the composition.

7. Composition according to one of the preceding claims, **characterised in that** the weight ratio between the extracts of boldo and the extracts of meadowsweet lies in the range 99:1 to 1:99, and preferably in the range 10:1 to 1:10.

8. Composition according to one of the preceding claims, formulated for use in the prevention, treatment or assisted control of microbial infections in animals.

9. Composition formulated for use according to claim 8, formulated for use in the prevention, treatment or assisted control of *Staphylococcus pseudintermedius* and/or *Malassezia pachydermatis* infections.

10. Composition formulated for use according to one of the preceding claims, formulated for use as a stimulator of the production of antimicrobial peptides.

11. Composition formulated for use according to claim 10, **characterised in that** the antimicrobial peptides are cathelicidins and/or beta-defensins.

12. Composition or composition formulated for use according to one of the preceding claims, **characterised in that** it does not stimulate the production of pro-inflammatory cytokines.

13. Composition or composition formulated for use according to any of the preceding claims, **characterised in that** the animal has an alteration of the skin barrier.

14. Composition or composition formulated for use according to claim 13, **characterised in that** the animal suffers from skin lesions caused by dermatitis (*Malassezia* dermatitis, atopic dermatitis), pyoderma, chronic otitis or fungal or bacterial infections.

15. Non-therapeutic use of a composition according to any of claims 1 to 7 as a skin disinfectant.

16. Non-therapeutic use of a composition according to any of claims 1 to 7 as an ear cleaner.

17. Non-therapeutic method of stimulating antimicrobial peptide-producing cells such as cathelicidins and/or beta-defensins in an animal, **characterised in that** a composition according to any of claims 1 to 7 is applied to the animal.

18. Method of producing a composition according to one of claims 1 to 7, **characterised in that** a composition containing extracts of boldo *Peumus Boldus* and a composition containing extracts of meadowsweet *Spiraea ulmaria* are combined within the same formulation.
